# EUROPEAN PATENT SPECIFICATION

(11) **EP 1 289 580 B1**
(45) Date of publication and mention of the grant of the patent: **23.01.2008**
(21) Application number: 01937997.3
(22) Date of filing: 29.05.2001
(51) Int. Cl.: A61M 5/172, G01N 33/487

(54) **A MEDICATION DELIVERY DEVICE WITH REPLACEABLE COOPERATING MODULES**
ABGABEVORRICHTUNG FÜR MEDIKAMENTE MIT AUSWECHSELBAREN, ZUSAMMENWIRKENDEN MODULEN
DISPOSITIF D'ADMINISTRATION DE MEDICAMENT AVEC MODULES COOPERANT REMPLAçABLES

(30) Priority: 30.05.2000 DK 200000852
(43) Date of publication of application: 12.03.2003
(73) Proprietor: NOVO NORDISK A/S, 2880 Bagsvaerd (DK)
(72) Inventor: KLITMOSE, Lars, Peter, DK-2820 Gentofte (DK)
(86) International application number: PCT/DK2001/000373
(87) International publication number: WO 2001/091833

(56) References cited:
- WO-A1-95/24233
- WO-A1-99/59657
- US-A- 5 713 856
- US-A- 5 781 442

## Description

### The Technical Field of the Invention:

The invention relates to drug administration systems for medical self-treatment.

The invention relates specifically to: A medication delivery device.

### Description of Related Art:

For people who frequently have to take medicine using some sort of delivery device, different options as regards treatment may be convenient in different situations.

Further, for a producer of a medication delivery device, it is of importance to be able to customize the devices to the needs and wishes (including more design oriented 'functionality') of any potential buyer of the product and at the same time achieve economies of scale.

US-A-5, 925, 021 discloses a medication delivery device that uses a microprocessor and a display to record, analyze and visualize various data concerning the medication administration. A single, all-in-one device that performs a variety of functions is provided.

WO-A-99/59657 discloses a medical apparatus for use by a patient for medical self-treatment of diabetes. The various functional units are physically tied together in a compact device. During use, each functional unit is employed individually.

US-A-5,713,856 discloses a modular patient care system with supply of therapeutic requirements, comprising an interface unit and a plurality of patient functional units. Each interface unit contains interface ports connecting the modules and an advanced interface unit provides physical attachment of the system to structures such as IV poles and bed rails.

### Disclosure of the invention:

The problem of the prior art is that the multifunction devices provide more functions than are needed in a certain situation and may thus be complicated to operate or more voluminous or more expensive than necessary, etc. The devices comprising non-cooperating, physically coupled individual units that are employed individually may be handy to take with you and ensure that you bring the necessary functional units, but are inconvenient in use in that they have to be mechanically separated and handled independently.

The object of the present invention is to provide a handy medication delivery device that may be easily adjusted to the different needs of a given user in different situations and to the different needs of different users, and which is economic from a production point of view.

This is achieved with a portable medication delivery device according to claim 1. The invention comprises a basis module and one or more replaceable modules each of which is adapted to cooperate mechanically and/or electronically with the basis module to provide a specific function, and the basis module includes resources that are jointly used by the replaceable modules.

In the present context, the term 'medication delivery device' is taken to mean, an injector type device (such as a pen injector or a jet injector) for delivering a discrete dose of a liquid medication (possibly in the form of small drops), a medication pump for continuous delivery of a liquid medication, an inhaler, spray or the like for delivering a discrete or continuous dose of a medication in vaporized, 'atomized' or pulverized form.

In the present context, the term 'replaceable module' is taken to mean that said module may be conveniently added to or removed from the basis module according to the user's needs in a given situation. I.e. each replaceable module may be interchanged with a different replaceable module, which together with the basis module provides a different function. Further, a producer of medication delivery devices will be able to tailor several different 'models' of devices based upon the same basis module (or basis modules) by combining it with one or more replaceable modules from a collection of 'standard' modules and achieve the combined benefits of using standard components (or building blocks) and still being able to do it in an economic way. Further, due to the modular way of building the devices, each module having a standard mechanical and (if relevant) electrical interface to the basis module and to other replaceable modules, it is possible for a user to buy a minimum configuration device according to his or her present needs and then to 'upgrade' the device along the way.

The basis module comprises basic elements of a medication delivery device. Some of or all of the basic elements of the basis module may be utilized by the different replaceable modules to provide different functions.

The possibility to flexibly configure the medication delivery device according to need has the following combined advantages:
- It makes possible the fulfillment of the needs of an individual user originating from short-term changes such as use at home vs. use out of home and from longer-term changes due to changes in the treatment, development of new functional modules etc.
- It makes it possible to build the production of medication delivery devices around a limited number of functional modules. This provides a rational concept for economically delivering high quality, mass-produced devices capable of being customized to individual user's needs for functionality by avoiding the duplication of key components, while still keeping the modular structure of the device.

As the basis module at least comprises means for holding a medication cartridge, means for transferring a part of or all of a medication contained in said medication cartridge from said medication cartridge to a user, means for receiving one or more replaceable modules, and means for supplying electric energy to the basis module and to the replaceable modules, it is ensured that the basic components for delivering a dose to a user from a medication cartridge, including means for energizing the transfer of the medication to a user and for supplying electric energy to all other electrical components in the basis module and in the replaceable modules, are provided.

When the basis module further comprises electronic means for monitoring and controlling the medication delivery process and for communicating with replaceable modules and with the user, it is ensured that the individual functions may be electronically driven and controlled, which generally improves reliability and accuracy, and that data from different functional modules may be combined with user input to produce intelligent results and improve user comfort and safety, and that a history of the drug administration process may be generated.

In a preferred embodiment said medication cartridge is replaceable and has an outlet and a movable wall, which, when displaced in the direction of the outlet, forces the contents out of the cartridge through said outlet.

When the outlet of said medication cartridge is connected to a replaceable catheter, and said means for transferring medication to a user is adapted to work in a continuous mode, so that medication is forced out of the cartridge through the outlet of said catheter, it is ensured that a continuous dose profile may be delivered to a user.

In a preferred embodiment said means for transferring a part of or all of the medication from said medication cartridge to the user at least comprise a piston rod being operable to engage and displace said movable wall, electrically driven actuating means, and driving means for transferring movement from said electrically driven actuating means to said piston rod.

When said means for receiving the replaceable modules comprise means for mechanically receiving and fixing said replaceable modules to the basis module, and means for electrically connecting said replaceable modules to electronic means of the basis module and to said means for supplying electric energy, it is ensured that a 'standard' interface for coupling the replaceable modules mechanically and electrically to the basis module is provided. If the energizing means are provided in the form of a battery pack, it is ensured that the device may be used when 'on the move', and that the supply of energy may be provided using standard battery techniques (based on rechargeable or non-rechargeable batteries) as e.g. developed for mobile telephones.

In a preferred embodiment electronic means for monitoring and controlling the medication delivery process and for communicating with replaceable modules and with the user are contained in a replaceable module themselves.

When said electronic means at least comprise means for controlling a delivered dose by controlling the displacement of the movable wall with said piston rod, through a control of the electrically driven actuating means via the driving means for transferring movement from said electrically driven actuating means to said piston rod, and means for monitoring the volume of delivered medication corresponding to said displacement of said movable wall, means for inputting data from the user, memory means for storing data, means for communicating with the replaceable modules, means for controlling the function of the basis module and the replaceable modules, processing means for processing input data, for processing data received from the replaceable modules and for processing data stored in said memory means, and a display for visualizing said data, it is ensured that the delivered dose is electronically controlled allowing an improved accuracy compared to a purely mechanical solution, and that a register of the used medication over time and the currently remaining volume of medication in the cartridge, etc. may be generated. It further ensures that information from the various functional modules of the delivery device and from the user input may be centrally stored and analyzed and that control signals for each individual replaceable module may be transferred on the basis hereof and relevant information be presented to the user. It further ensures that a check of the correct function of each replaceable module of the delivery device and the cooperation with the basis module may be performed and that an alarm may be issued or the intended action blocked in case the result of the check is negative.

When said electronic means include means for reading an item of information on a replaceable medication cartridge when said cartridge is placed in said means for holding a replaceable medication cartridge, and means for processing said item of information, it is ensured that the process of reading and checking the contents of the medication cartridge may be automated resulting in a higher degree of safety in the handling of the medication.

When said electronic means are adapted to receive a user-specific unit containing user data, functional check procedures and user authorizing procedures, it is ensured that a check of the correct function of each replaceable module of the delivery device and the cooperation with the basis module and a check of the user ID to prevent unauthorized use of the delivery device may be provided in a single, possibly exchangeable unit customized to the actual user specific data, the present configuration of the device, and optionally containing data indicating which functionality the user has a license to. Alternatively, the processing means may be adapted to identify the present configuration of the device in terms of basis module and replaceable modules including software, when the power is turned on.

In a preferred embodiment said user-specific unit is a chip card.

When the replaceable modules may be chosen from a group consisting of
- a replaceable module containing a system for blood glucose monitoring;
- a replaceable module containing a system for continuously measuring blood glucose;
- a replaceable module containing a modem for allowing communication with a data communications network;
- a replaceable module containing a communications interface for wireless communication with other devices;
- a replaceable module containing fixed wire interfaces for communication with one or more of a personal computer, a camera, a TV-monitor, an acoustic device, a telephone, a mobile telephone;
- a replaceable module containing the functionality of a mobile telephone;
- a replaceable module containing a loudspeaker;
- a replaceable module containing a microphone, a loudspeaker and a processor and software for speech recognition for providing a voice interface;
- a replaceable module containing means for monitoring the temperature of the medication cartridge and its contents;
- a replaceable module containing means for monitoring and controlling the temperature of the medication cartridge and its contents;
- a replaceable module containing means for providing a selectable acoustic or vibratory or optical signal after a certain settable time or on the occurrence of a certain event;
- a replaceable module containing means for vibrating the contents of the medication cartridge, and means for providing an alarm signal indicating the elapsing of a settable time to ensure a proper mixing of the constituents of the medication cartridge;
- a replaceable module containing means for detecting shaking movements of the medication delivery device and means for providing an alarm signal indicating that a certain amount of shaking movements has been performed to ensure a proper mixing of the constituents of the medication cartridge;
- a replaceable module containing software for controlling the medication delivery at settable velocities, controlled time scales, maximum delivered doses, etc.;
- a replaceable module containing software for generating a log of certain user defined events monitored by the medication delivery device;
- a replaceable module containing software for controlling a user ID;
- a replaceable module containing a display adapted for left-handed use;
- a replaceable module containing a display adapted for right-handed use;
- a replaceable module containing means for delivering a specific dose profile to a user through a catheter by controlling said means for transferring the medication in such a way that a continuous pump mode is provided,
it is ensured that the medication delivery device may be configured to a wide range of functionally different devices based upon standardized building blocks.

When the basis module and the replaceable modules are provided with replaceable covers, it is ensured that the design or look of the medication devices may be adjusted to the instant user wishes, e.g. to match a dress or the like.

When the basis module contains functionality that may be locked and made available to the user only by a unique software key and/or a software update, it is ensured that several functions may be provided in a single module, if appropriate, the use of each function being dependent of the input of a specific key word and/or a software update. This could be of relevance in connection with certain functions that are related/overlapping in hardware and or/software implementation.

A method of making a medication delivery device is furthermore presented. When it comprises the steps of
- (a) defining and constructing a basis module containing common resources, and
- (b) defining and constructing one or more replaceable modules each of which being adapted to cooperate mechanically and electronically with the basis module to provide a specific function, and
- (c) deciding a configuration of functions according to need, based on a selection of possible functions, and
- (d) composing a device implementing the decided functions by combining the relevant basic module and one or more replaceable modules,
- possibly repeating steps (c) and (d), in case of changing functionality needs,
the same advantages as disclosed above for claim 1 are achieved.

When the steps of deciding a configuration of functions according to need and composing a device implementing the decided functions are performed by a user of the device, it is ensured that the fulfillment of the needs of an individual user originating from short-term changes such as use at home vs. use out of home and from longer-term changes due to changes in the treatment, development of new functional modules etc. are made possible.

When the steps of deciding a configuration of functions according to need and composing a device implementing the decided functions are performed by a producer or supplier of the device, it is possible to build the production of differently configured medication delivery devices around a limited number of functional modules. This provides a rational concept for economically delivering high quality, mass-produced devices capable of being customized to individual user's needs for functionality by avoiding the duplication of key components.

When it further comprises the step of locking the device so that it cannot be separated into its constituent modules by a user, the responsibility for a correct function of the final device as delivered may be guaranteed by the producer/supplier of the device without performing special check routines prior to each use of the device.

### Brief Description of the Drawings:

The invention will be explained more fully below in connection with a preferred embodiment and with reference to the drawings in which:
fig. 1 shows an injection device according to the invention,
fig. 2 shows a preferred embodiment of a basis module according to the invention including basic resources for dosing,
fig. 3 shows a preferred embodiment of a basis module according to the invention including basic resources for dosing as well as electronic processing means,
fig. 4 shows electronic means of a basis module for controlling the medication delivery process and for communicating with the replaceable modules and with a user,
fig. 5 shows a selection of replaceable modules according to the invention, and
fig. 6 shows a preferred embodiment of the method presented.

The figures are schematic and simplified for clarity, and they just show details, which are essential to the understanding of the invention, while other details are left out. Throughout, the same reference numerals are used for identical or corresponding parts.

### Detailed Description of Embodiments:

A module based on an electromechanical unit for moving a piston rod to move a piston in a replaceable cartridge with an outlet and a processing unit for controlling the medication delivery process etc. together with I/O-components is the base unit in the following embodiments of the invention on which different replaceable modules can be attached to give different applications, such as BGM, voice interface for visually disabled persons, etc.

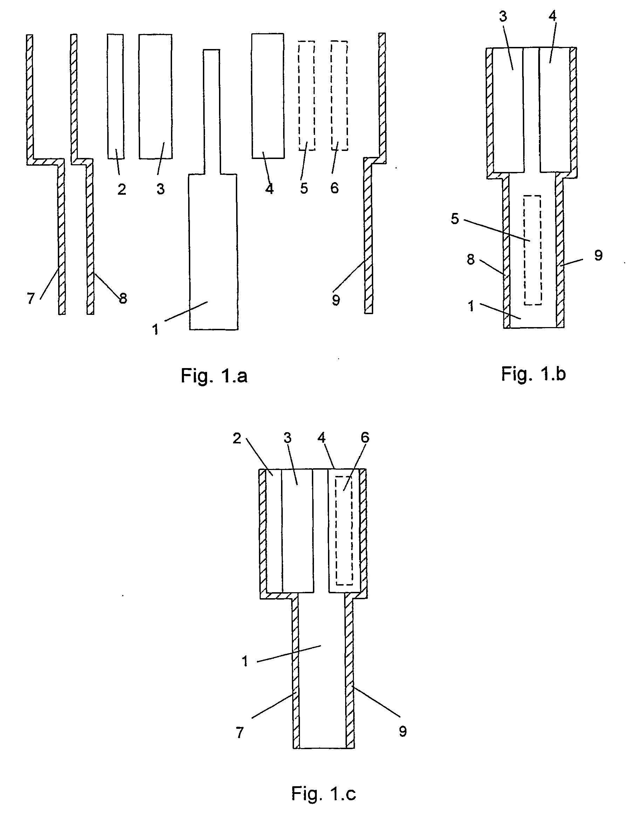

Fig. 1 shows an injection device according to the invention illustrating its modular construction.

Fig. 1.a shows the basis module 1 containing basic mechanical and electrical resources necessary for the delivery process and the control hereof and various replaceable modules 2, 3, 4, 5, 6 each of which, together with the resources of the basis module, implement a specific function. The replaceable modules may consist of hardware (and optionally software) 2, 3, 4 or be pure software modules 5, 6. Special cover modules for defining the visual impression of the medication delivery device are shown 7, 8, 9. The covers may have different colors, be made of different materials have different surfaces and forms. Covers may be mounted during production so that the device comes with standard or pre-selected covers, which may then later be replaced with other covers according to the user's preferences. The materials of the covers may have different special properties, e.g. elastic or luminescent or water repellant etc. The covers may constitute a water-resistant or watertight enclosure of the medication delivery device.

In fig. 1.b a medication delivery device consisting of a basis module 1, two replaceable hardware modules 3, 4, and a software module 5 loaded into a memory of the basis module is shown. The device is finished with cover modules 8, 9.

In fig. 1.c a medication delivery device consisting of a basis module 1, three replaceable hardware modules 2, 3, 4, and a software module 6 loaded into a memory of replaceable module 4 is shown. The device is finished with cover modules 7, 9.

The medication delivery devices in figs. 1.b and 1.c may be built together by the user of the device according to his or her present needs or alternatively by the supplier of the device.

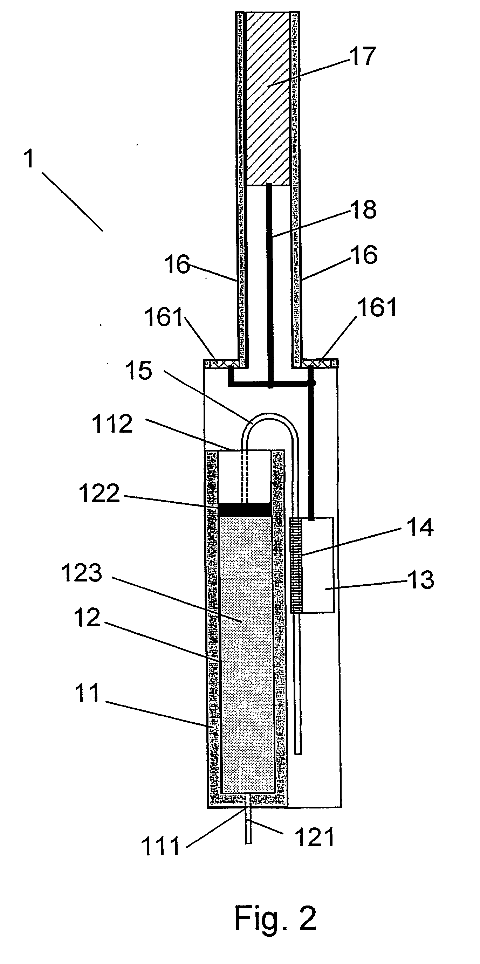

Fig. 2 shows a preferred embodiment of a basis module according to the invention including basic resources for dosing.

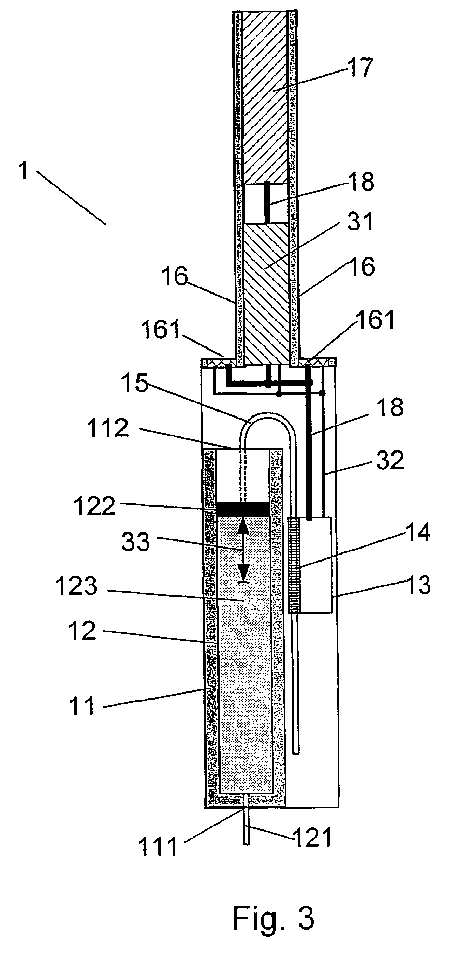

Fig. 2 shows an embodiment of a basis module 1 for an injection device of the pen type according to the invention, in which a cylindrical replaceable medication cartridge 12 having an outlet 121 at one end and a lid that is formed as a piston 122 at the opposite end is placed in corresponding receiving means 11. The receiving means have an opening 111 at one end for the outlet 121 of the cartridge 12 and an opening 112 at the other end for the piston rod 15. The piston rod, which is adapted to interact with the piston to displace the piston, is formed with a 180 degree bend to allow a more compact construction. It might as well, however, be implemented as a normal straight piston rod. An electric motor 13, powered from a battery or battery pack 17 via electrical conductors 18 engage with driving means 14 and corresponding driving means (not shown) on the piston rod to displace the piston rod in its axial direction, thereby displacing the piston and forcing the medication out of the cartridge through the outlet 121. In fig. 2, the basis module is adapted for an injection device of the pen type, the outlet taking the form of a disposable needle, but it might as well take the form of a small disposable tube in the case of a jet injector. The piston rod 15 is cylindrical, axially stiff, but radially salient and provided with a thread (not shown) that together with a corresponding driving nut provided with a gear wheel (not shown) on its outer periphery and a corresponding cooperating gear wheel (not shown) on the motor constitute the driving means 14 for transferring movement from the motor 13 to the piston rod 15. The means 16 for receiving the replaceable modules (e.g. 2, 3, 4 of fig. 1) include electric interfaces 161 in the form of one or more connectors for power feeding the replaceable modules via electrical conductors 18 and for allowing the transfer of data between the basis module and the replaceable modules (cf. fig. 3).

Fig. 3 shows a preferred embodiment of a basis module according to the invention including basic resources for dosing as well as electronic processing means.

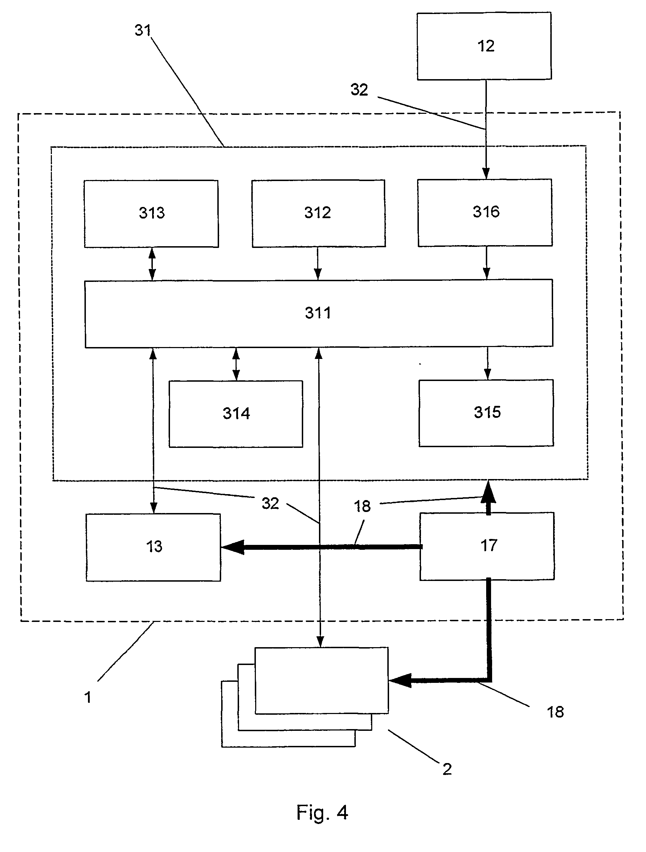

In addition to the features of fig. 2, fig. 3 contains electronic means 31 for monitoring and controlling the medication process and for communicating with the replaceable modules (e.g. 2, 3, 4 of fig. 1) and with a user. Electrical wire connections 32 for transmitting and receiving signals to and from the replaceable modules and the electric motor are included from the electronic means 31 to the electric interfaces 161 to the replaceable modules and to the motor. The electronic means 31 include processing means (311 on fig. 4) that are adapted to control the process of delivering a precise dose according to a given specification by controlling the displacement 33 of the piston through an accurate displacement of the piston rod by means of the electric motor 13 and the driving means 14.

Fig. 4 shows electronic means of a basis module for controlling the medication delivery process and for communicating with the replaceable modules and with a user.

The electronic means 31 of a basis module according to the invention comprise a processor 311, memory means 313 (volatile (e.g. RAM) as well as non-volatile), a display 315 for showing user inputs and processed results to the user, means 316 for reading an item of information on a medication cartridge 12, a user input device 312 (e.g. a, possibly limited, keyboard) for inputting data from a user and a control unit 314 containing user-specific data and optionally functional check procedures and a user authorization procedure, possibly in the form of a chip card (like a SIM card of a mobile telephone). The battery 17 and its connections 18 for powering the electronic means 31 and the electric motor 13 and the replaceable modules 2 are also shown in fig. 4 together with the signal wires 32 for communication between the processor 311, the motor 13 and the replaceable modules and for transferring an item of information from a cartridge 12 to a corresponding reading device 316.

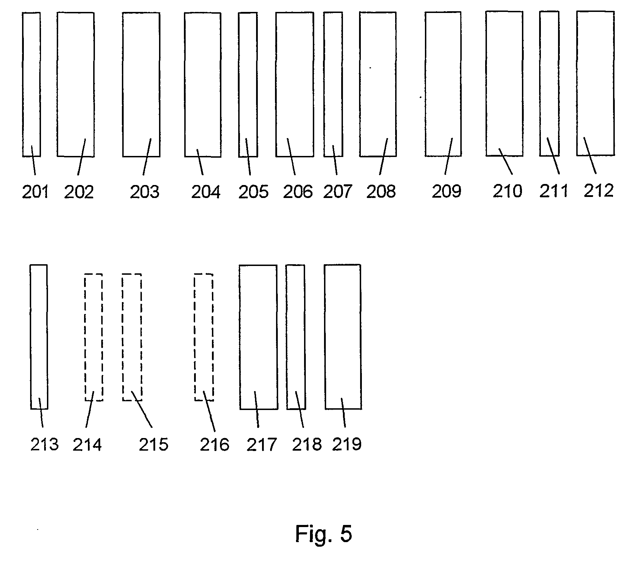

Fig. 5 shows a selection of replaceable modules according to the invention.

Fig. 5 sketches various replaceable modules from which a medication delivery device according to the invention may be composed in combination with a basis module (cf. 1 in figs. 1-3). The replaceable modules, which are briefly described in the following with reference to fig. 5, only represent examples and does not provide an exhaustive list of relevant modules within the scope of the invention:

A replaceable module 201 containing a system for blood glucose monitoring.

A replaceable module 202 containing a system for continuously measuring blood glucose.

A replaceable module 203 containing a modem for allowing communication with a data communications network such as the Internet or any other local or global data communications network. This module may be used for downloading software to the device, for remote check of the functionality of the device, for remote diagnostics, for enabling user authorization, etc.

A replaceable module 204 containing a communications interface for wireless communication with other devices. This module implements one or more of the standards for wirelessly communicating with other devices, e.g. the Bluetooth-standard, a wireless modem, infrared communication, etc.

A replaceable module 205 containing fixed wire interfaces for communication with one or more of a personal computer, a camera, a TV-monitor, an acoustic device, a telephone, a mobile telephone. The module includes the relevant electrical connector interfaces.

A replaceable module 206 containing the functionality of a mobile telephone.

A replaceable module 207 containing a loudspeaker.

A replaceable module 208 containing a microphone, a loudspeaker and a processor and software for speech recognition for providing a voice interface. This module implements a voice interface, e.g. for visually disabled persons.

A replaceable module 209 containing means for monitoring the temperature of the medication cartridge and its contents. This module is e.g. aimed at providing a user with information about the minimum and maximum temperatures, to which the currently loaded medication cartridge has been exposed, in order to decide whether it is usable.

A replaceable module 210 containing means for monitoring and controlling the temperature of the medication cartridge and its contents. This module is e.g. aimed at ascertaining that the currently loaded medication is usable, irrespective of the temperatures that the device experiences (within certain limits).

A replaceable module 211 containing means for providing a selectable acoustic or vibratory or optical signal after a certain settable time or on the occurrence of a certain event. This module is aimed at helping the user to observe a certain given pattern of treatment over time.

A replaceable module 212 containing means for vibrating the contents of the medication cartridge, and means for providing an alarm signal indicating the elapse of a settable time to ensure a proper mixing of the constituents of the medication cartridge.

A replaceable module 213 containing means for detecting shaking movements of the medication delivery device and means for providing an alarm signal indicating that a certain number of shaking movements has been performed to ensure a proper mixing of the constituents of the medication cartridge.

A replaceable module 214 containing software for controlling the medication delivery at settable velocities, controlled time scales (for possible use with replaceable module 211), maximum delivered doses, etc. The module is aimed at controlling the medication delivery process as regards the speed profile of the delivery, the (minimum and maximum) time between deliveries, the volume of the delivered dose, etc.

A replaceable module 215 containing software for generating a log of certain user defined events monitored by the medication delivery device. Such relevant events are time of drug deliveries, corresponding volumes, possibly temperature of the medication, possibly user inputs of relevant information to a given delivery (physical/mental stress, etc.).

A replaceable module 216 containing software for controlling a user ID by requiring the user to input a predefined sequence of characters to prevent unauthorized use of the device.

A replaceable module 217 containing a display adapted for left-handed use.

A replaceable module 218 containing a display adapted for right-handed use.

A replaceable module 219 containing means for delivering a specific dose profile to a user through a catheter having a needle at one end and whose other end is connected to the outlet of the medication cartridge, by controlling the means for transferring the medication in such a way that a continuous pump mode is provided. This module is aimed at a situation where a delivery of medication is required over a certain amount time (as opposed to an injection-type delivery, being typically of a duration of a few seconds).

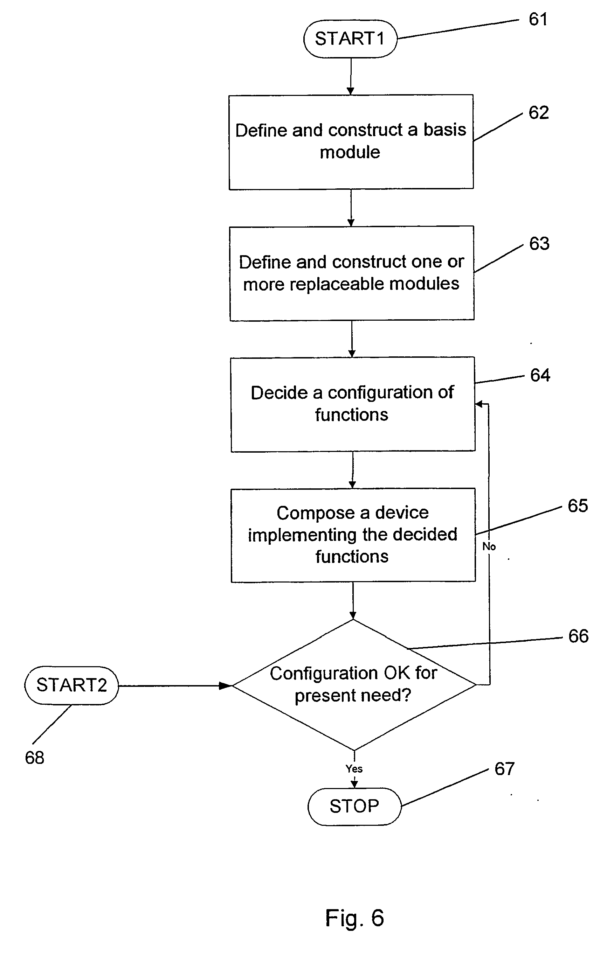

Fig. 6 shows a preferred embodiment of the method presented.

Fig. 6 illustrates a method of making a medication delivery device in a modular fashion. The process is started 61 by performing the step 62 of defining and constructing a basis module containing basic components and common resources such as described in claims 2-12 and as illustrated in figs. 1-4. In parallel hereto or subsequently, the step 63 of defining and constructing one or more replaceable modules, each of which being adapted to cooperate mechanically and electronically with the basis module to provide a specific function, is performed. Examples of such replaceable modules are disclosed in claim 13 and discussed above in connection with fig. 5. Subsequently, the step 64 of deciding a configuration of functions according to need, based on a selection of possible functions (e.g. among those represented by the replaceable modules just mentioned) is performed. This step may be carried out by a user sorting out a relevant configuration for a given situation or by a producer or supplier of the devices in the process of defining the relevant functions of devices to fulfill the needs of a specific customer segment. Subsequently, the step 65 of composing or building a device implementing the decided functions by combining the relevant basic module and one or more replaceable modules (such as those outlined above and sketched in fig. 5) is performed.

If a user has a collection of replaceable modules for implementing a variety of functions, a step 66 of deciding whether the present configuration serves the present needs may be performed. If OK, the process is stopped 67, and if the present configuration is not suitable, steps 64 and 65 are repeated. The latter process of deciding whether the present configuration serves the present needs may alternatively be initiated from point 68, which represent a normal case of a user wondering whether the device as it is conforms to the requirements of the situation.

If the device is assembled by a producer or supplier of the device, it may be of interest to ensure that the user is not able to detach the modules and assemble them again (by mechanically or electronically 'lock' them together) in order for the producer or supplier to be able to guarantee the correct function of the device. Alternatively, a check procedure may be implemented by each power up of the device.

Some preferred embodiments have been shown in the foregoing, but it should be stressed that the invention is not limited to these, but may be embodied in other ways within the subject matter defined in the following claims. For example, above the electronic means for controlling the medication delivery process, etc., were part of a basis module. It might as well be part of a replaceable module, possibly, if convenient and/or economical, divided in several replaceable modules. Likewise, in the above embodiments only one basis module is referred to. However, one of a selection of several different basis modules may form the core of the medication delivery device (e.g. including more or less basic electronics such as electro-acoustic interface, special power supplies etc.).

## Claims

1. A portable medication delivery device
comprising a basis module with basic elements for dosing (1) and one or more replaceable modules (2, 3, 4, 5, 6), each of the said modules comprises a standard mechanical and an electrical interface to said basis module and/or to other replaceable modules, such that each module may be conveniently interchanged with a different module, thereby providing different additional function; the basis module (1) at least comprises means for holding (11) a medication cartridge (12), means for transferring (13, 14, 15) a part of or all of a medication (123) contained in said medication cartridge (12) from said medication cartridge to a user, means for receiving (16) one or more replaceable modules (2, 3, 4, 5, 6), wherein the basis module (1) comprises means for supplying (17, 18) electric energy to the basis module and to the replaceable modules.

2. A portable medication delivery device according to claim 1,
**characterized in that**
the basis module (1) further comprises electronic means (31) for monitoring and controlling the medication delivery process and for communicating with replaceable modules and with the user.

3. A portable medication delivery device according to claim 1 or 2,
**characterized in that**
said medication cartridge (12) is replaceable and has an outlet (121) and a movable wall (122), which, when displaced in the direction of the outlet (121), forces the contents (123) out of the cartridge (12) through said outlet (121).

4. A portable medication delivery device according to claim 3,
**characterized in that**
the outlet (121) of said medication cartridge (12) is connected to a replaceable catheter, and said means for transferring (13, 14, 15) medication to a user is adapted to work in a continuous mode, so that medication (123) is forced out of the cartridge (12) through the outlet of said catheter.

5. A portable medication delivery device according to any one of claims 1-3,
**characterized in that**
said means for transferring (13, 14, 15) a part of or all of the medication (123) from said medication cartridge (12) to the user at least comprise a piston rod (15) being operable to engage and displace said movable wall (122), electrically driven actuating means (13), and driving means (14) for transferring movement from said electrically driven actuating (13) means to said piston rod (15).

6. A portable medication delivery device according to any one of claims 1-5,
**characterized in that**
said means for receiving (16) the replaceable modules comprise means for mechanically receiving and fixing said replaceable modules to the basis module, and means for electrically connecting (161) said replaceable modules (2, 3, 4, 5, 6) to electronic means (31, 32) of the basis module (1) and to said means for supplying (17, 18) electric energy.

7. A portable medication delivery device according to any one of claims 2-6,
**characterized in that**
electronic means for monitoring and controlling (31, 32) the medication delivery process and for communicating with replaceable modules and with the user are contained in a portable replaceable module themselves.

8. A portable medication delivery device according to any one of claims 2-7,
**characterized in that**
said electronic means (31, 32) at least comprise means for controlling a delivered dose by controlling the displacement (33) of the movable wall (122) with said piston rod (15), through a control of the electrically driven actuating means (13) via the driving means (14) for transferring movement from said electrically driven actuating means (13) to said piston rod (15), and means for monitoring the volume of delivered medication corresponding to said displacement (33) of said movable wall (122), means for inputting (312) data from the user, memory means (313) for storing data, means for communicating (311, 32) with the replaceable modules (2, 3, 4, 5, 6), means for controlling (314) the function of the basis module and the replaceable modules, processing means (311) for processing input data, for processing data received from the replaceable modules and for processing data stored in said memory means, and a display (315) for visualizing said data.

9. A portable medication delivery device according to any one of claims 2-8,
**characterized in that**
said electronic means (31) include means for reading (316) an item of information on a replaceable medication cartridge (12) when said cartridge is placed in said means for holding (11) a replaceable medication cartridge, and means for processing (311) said item of information.

10. A portable medication delivery device according to any one of claims 2-9,
**characterized in that**
said electronic means are adapted to receive a user-specific unit (314) containing user data, functional check procedures and user authorizing procedures.

11. A portable medication delivery device according to claim 10,
**characterized in that**
said user-specific unit (314) is a chip card.

12. A portable medication delivery device according to any one of claims 1-11,
**characterized in that**
the replaceable modules may be chosen from a group consisting of
• a replaceable module (201) containing a system for blood glucose monitoring;
• a replaceable module (202) containing a system for continuously measuring blood glucose;
• a replaceable module (203) containing a modem for allowing communication with a data communications network;
• a replaceable module (204) containing a communications interface for wireless communication with other devices;
• a replaceable module (205) containing fixed wire interfaces for communication with one or more of a personal computer, a camera, a TV-monitor, an acoustic device, a telephone, a mobile telephone;
• a replaceable module (206) containing the functionality of a mobile telephone;
• a replaceable module (207) containing a loudspeaker;
• a replaceable module (208) containing a microphone, a loudspeaker and a processor and software for speech recognition for providing a voice interface;
• a replaceable module (209) containing means for monitoring the temperature of the medication cartridge and its contents;
• a replaceable module (210) containing means for monitoring and controlling the temperature of the medication cartridge and its contents;
• a replaceable module (211) containing means for providing a selectable acoustic or vibratory or optical signal after a certain settable time or on the occurrence of a certain event;
• a replaceable module (212) containing means for vibrating the contents of the medication cartridge, and means for providing an alarm signal indicating the elapsing of a settable time to ensure a proper mixing of the constituents of the medication cartridge;
• a replaceable module (213) containing means for detecting shaking movements of the medication delivery device and means for providing an alarm signal indicating that a certain amount of shaking movements has been performed to ensure a proper mixing of the constituents of the medication cartridge;
• a replaceable module (214) containing software for controlling the medication delivery at settable velocities, controlled time scales, maximum delivered doses, etc.;
• a replaceable module (215) containing software for generating a log of certain user defined events monitored by the medication delivery device;
• a replaceable module (216) containing software for controlling a user ID;
• a replaceable module (217) containing a display adapted for left-handed use;
• a replaceable module (218) containing a display adapted for right-handed use;
• A replaceable module (219) containing means for delivering a specific dose profile to a user through a catheter by controlling said means for transferring the medication in such a way that a continuous pump mode is provided.

13. A portable medication delivery device according to any one of claims 1-12,
**characterized in that**
the basis module and the replaceable modules are provided with replaceable covers (7, 8, 9).

14. A portable medication delivery device according to any one of claims 1-13,
**characterized in that**
the basis module contains functionality that may be locked and made available to the user only by a unique software key and/or a software update.

## Patentansprüche

1. Tragbare Medikamentenabgabevorrichtung
umfassend ein Basismodul mit Basiselementen zur Dosierung (1) und ein oder mehrere austauschbare Module (2, 3, 4, 5, 6), wobei jedes der austauschbaren Module eine standardisierte mechanische und eine elektrische Schnittstelle am Basismodul und/oder an anderen austauschbaren Module umfasst, sodass jedes Modul bequem mit unterschiedlichen Modulen ausgetauscht werden kann, wodurch unterschiedliche zusätzliche Funktionen bereitgestellt werden; wobei das Basismodul (1) mindestens ein Mittel zum Halten (11) einer Medikamentenpatrone (12), Mittel zum Übertragen (13, 14, 15) eines Teils oder des gesamten eines in der Medikamentenpatrone (12) enthaltenen Medikaments (123) auf einen Anwender, ein Mittel zum Aufnehmen (16) von einem oder mehreren austauschbaren Modulen (2, 3, 4, 5, 6) umfasst, wobei das Basismodul (1) Mittel zum Zuführen (17, 18) von elektrischer Energie zum Basismodul und zu den austauschbaren Modulen umfasst.

2. Tragbare Medikamentenabgabevorrichtung nach Anspruch 1,
**dadurch gekennzeichnet, dass**
das Basismodul (1) des Weiteren ein elektronisches Mittel (31) zum Überwachen und Steuern des Medikamentenabgabevorgangs und zum Kommunizieren mit austauschbaren Modulen und mit dem Anwender umfasst.

3. Tragbare Medikamentenabgabevorrichtung nach Anspruch 1 oder 2,
**dadurch gekennzeichnet, dass**
die Medikamentenpatrone (12) austauschbar ist und einen Auslass (121) und eine bewegliche Wand (122) aufweist, die, wenn sie in die Richtung des Auslasses (121) verschoben wird, den Inhalt (123) durch den Auslass (121) aus der Patrone (12) zwingt.

4. Tragbare Medikamentenabgabevorrichtung nach Anspruch 3,
**dadurch gekennzeichnet, dass**
der Auslass (121) der Medikamentenpatrone (12) mit einem austauschbaren Katheter verbunden ist und das Mittel zum Übertragen (13, 14, 15) von Medikament auf einen Anwender angepasst ist, um in einem kontinuierlichen Modus zu arbeiten, sodass das Medikament (123) durch den Auslass des Katheters aus der Patrone (12) gezwungen wird.

5. Tragbare Medikamentenabgabevorrichtung nach einem der Ansprüche 1 - 3,
**dadurch gekennzeichnet, dass**
das Mittel zum Übertragen (13, 14, 15) eines Teils des oder des gesamten Medikaments (123) aus der Medikamentenpatrone (12) auf den Anwender mindestens eine Kolbenstange (15), die bedienbar ist, um in die bewegbare Wand (122) einzugreifen und sie zu verschieben, ein elektrisch angetriebenes Betätigungsmittel (13) und ein Antriebsmittel (14) zum Übertragen von Bewegung vom elektrisch angetriebenen Betätigungsmittel (13) auf die Kolbenstange (15) umfasst.

6. Tragbare Medikamentenabgabevorrichtung nach einem der Ansprüche 1 - 5,
**dadurch gekennzeichnet, dass**
das Mittel zur Aufnahme (16) der austauschbaren Module Mittel zur Aufnahme und zum Fixieren der austauschbaren Module am Basismodul und Mittel zum elektrischen Anschließen (161) der austauschbaren Module (2, 3, 4, 5, 6) an elektrische Mittel (31, 32) des Basismoduls (1) und am Mittel zum Zuführen (17, 18) von elektrischer Energie umfasst.

7. Tragbare Medikamentenabgabevorrichtung nach einem der Ansprüche 2 - 6,
**dadurch gekennzeichnet, dass**
elektronische Mittel zum Überwachen und Steuern (31, 32) des Medikamentenabgabevorgangs und zum Kommunizieren mit austauschbaren Modulen und mit dem Anwender in einem tragbaren austauschbaren Modul selbst enthalten sind.

8. Tragbare Medikamentenabgabevorrichtung nach einem der Ansprüche 2 - 7,
**dadurch gekennzeichnet, dass**
die elektronischen Mittel (31, 32) mindestens Mittel zum Steuern einer Abgabedosis durch Steuern der Verschiebung (33) der beweglichen Wand (122) mit der Kolbenstange (15) durch eine Steuerung des elektrisch angetriebenen Betätigungsmittels (13) über das Antriebsmittel (14) zum Übertragen von Bewegung vom elektrisch angetriebenen Betätigungsmittel (13) auf die Kolbenstange (15) und Mittel zum Überwachen des Volumens des abgegebenen Medikaments, das der Verschiebung (33) der beweglichen Wand (122) entspricht, Mittel zum Eingeben (312) von Daten vom Anwender, Speichermittel (313) zum Speichern von Daten, Mittel zum Kommunizieren (331, 32) mit den austauschbaren Modulen (2, 3, 4, 5, 6), Mittel zum Steuern (314) der Funktion des Basismoduls und der austauschbaren Module, Verarbeitungsmittel (311) zum Verarbeiten von Eingabedaten zum Verarbeiten von aus den austauschbaren Modulen erhaltenen Daten und zum Verarbeiten von im Speichermittel gespeicherten Daten und eine Anzeige (315) zum Visualisieren der Daten, umfassen.

9. Tragbare Medikamentenabgabevorrichtung nach einem der Ansprüche 2 - 8,
**dadurch gekennzeichnet, dass**
die elektronischen Mittel (31) Mittel zum Lesen (316) eines Informationspunkts auf einer austauschbaren Medikamentenpatrone (12), wenn die Patrone in das Mittel zum Halten (11) einer austauschbaren Medikamentenpatrone angeordnet wird, und Mittel zum Verarbeiten (311) des Informationspunkts einschließen.

10. Tragbare Medikamentenabgabevorrichtung nach einem der Ansprüche 2-9,
**dadurch gekennzeichnet, dass**
die elektronischen Mittel angepasst sind, um eine anwenderspezifische Einheit (314) aufzunehmen, die Anwenderdaten, Funktionsüberprüfungsverfahren und Anwenderberechtigungsverfahren enthält.

11. Tragbare Medikamentenabgabevorrichtung nach Anspruch 10,
**dadurch gekennzeichnet, dass**
die anwenderspezifische Einheit (314) eine Chipkarte ist.

12. Tragbare Medikamentenabgabevorrichtung nach einem der Ansprüche 1 - 11,
**dadurch gekennzeichnet, dass**
die austauschbaren Module aus einer Gruppe ausgewählt sein können, bestehend aus
- einem austauschbaren Modul (201), enthaltend ein System zur Blutzuckerüberwachung;
- einem austauschbaren Modul (202), enthaltend ein System zur kontinuierlichen Messung des Blutzuckers;
- einem austauschbaren Modul (203), enthaltend ein Modem zum Ermöglichen von Kommunikation mit einem Datenkommunikationsnetzwerk;
- einem austauschbaren Modul (204), enthaltend eine Kommunikationsschnittstelle zur drahtlosen Kommunikation mit anderen Vorrichtungen;
- einem austauschbaren Modul (205), enthaltend fixierte Drahtschnittstellen zur Kommunikation mit einem oder mehreren PCs, einer Kamera, einem TV-Monitor, einer akustischen Vorrichtung, einem Telefon, einem Mobiltelefon;
- einem austauschbaren Modul (206), enthaltend die Funktionsweise eines Mobiltelefons;
- einem austauschbaren Modul (207), enthaltend einen Lautsprecher;
- einem austauschbaren Modul (208), enthaltend ein Mikrofon, einen Lautsprecher und einen Prozessor und Software für Spracherkennung zum Bereitstellen einer Sprachschnittstelle;
- einem austauschbaren Modul (209), enthaltend Mittel zum Überwachen der Temperatur der Medikamentenpatrone und ihres Inhalts;
- einem austauschbaren Modul (210), enthaltend Mittel zum Überwachen und Steuern der Temperatur der Medikamentenpatrone und ihres Inhalts;
- einem austauschbaren Modul (211), enthaltend Mittel zum Bereitstellen eines wählbaren akustischen oder vibrierenden oder optischen Signals nach einer bestimmten einstellbaren Zeit oder beim Auftreten eines bestimmten Ereignisses;
- einem austauschbaren Modul (212), enthaltend Mittel zum Schwenken des Inhalts der Medikamentenpatrone und Mittel zum Bereitstellen eines Alarmsignals, das das Verstreichen einer einstellbaren Zeit anzeigt, um ein richtiges Mischen der Bestandteile der Medikamentenpatrone sicher zu stellen;
- einem austauschbaren Modul (213), enthaltend Mittel zum Erkennen von Schüttelbewegungen der Medikamentenabgabevorrichtung und Mittel zum Bereitstellen eines Alarmsignals, das anzeigt, dass eine bestimmte Menge an Schüttelbewegungen durchgeführt wurde, um ein richtiges Mischen der Bestandteile der Medikamentenpatrone sicher zu stellen;
- einem austauschbaren Modul (214), enthaltend eine Software zum Steuern der Medikamentenabgabe bei einstellbaren Geschwindigkeiten, gesteuerten Zeitskalen, maximal abgegebener Dosen usw.;
- einem austauschbaren Modul (215), enthaltend eine Software zum Erzeugen eines logs an bestimmten anwenderdefinierten durch die Medikamentenabgabevorrichtung überwachten Ereignissen;
- einem austauschbaren Modul (216), enthaltend eine Software zum Steuern einer Anwender-ID;
- einem austauschbaren Modul (217), enthaltend eine für Linkshänderanwendung angepasste Anzeige;
- einem austauschbaren Modul (218), enthaltend eine für Rechtshänderanwendung angepasste Anzeige;
- einem austauschbaren Modul (219), enthaltend Mittel zum Abgeben eines spezifischen Dosisprofils an einen Anwender durch einen Katheter durch Steuern des Mittels zum Übertragen des Medikaments in einer derartigen Weise, dass ein kontinuierlicher Pumpmodus bereitgestellt ist

13. Tragbare Medikamentenabgabevorrichtung nach einem der Ansprüche 1 - 12,
**dadurch gekennzeichnet, dass**
das Basismodul und die austauschbaren Module mit austauschbaren Abdeckungen (7, 8, 9) bereitgestellt sind:

14. Tragbare Medikamentenabgabevorrichtung nach einem der Ansprüche 1 - 13,
**dadurch gekennzeichnet, dass**
das Basismodul eine Funktionsweise enthält, die verriegelt werden kann und für den Anwender nur durch einen einzigartigen Softwareschlüssel und/oder einer Softwareaktualisierung verfügbar gemacht wird.

## Revendications

1. Dispositif portatif d'administration de médicament comprenant un module de base ayant des éléments de base pour le dosage (1) et un ou plusieurs modules pouvant être remplacés (2, 3, 4, 5, 6), chacun desdits modules comprend une interface standard mécanique et électrique pour ledit module de base et/ou pour d'autres modules pouvant être remplacés, de sorte que chaque module peut être échangé de façon commode avec un module différent, fournissant de ce fait une fonction supplémentaire différente ; le module de base (1) comprend au moins un moyen pour maintenir (11) une cartouche de médicament (12), un moyen pour transférer (13, 14, 15) une partie ou la totalité d'un médicament (123) contenu dans ladite cartouche de médicament (12) depuis ladite cartouche de médicament vers un utilisateur, un moyen pour recevoir (16) un ou plusieurs modules pouvant être remplacés (2, 3, 4, 5, 6), dans lequel le module de base (1) comprend un moyen pour fournir (17, 18) de l'énergie électrique au module de base et aux modules pouvant être remplacés.

2. Dispositif portatif d'administration de médicament selon la revendication 1, **caractérisé en ce que** le module de base (1) comprend en outre un moyen électronique (31) pour contrôler et commander le processus d'administration de médicament et pour communiquer avec des modules pouvant être remplacés et avec l'utilisateur.

3. Dispositif portatif d'administration de médicament selon la revendication 1 ou 2, **caractérisé en ce que** ladite cartouche de médicament (12) peut être remplacée et possède une sortie (121) et une paroi mobile (122), qui, lorsqu'elle se déplace dans le sens de la sortie (121), force le contenu (123) hors de la cartouche (12) à travers ladite sortie (121).

4. Dispositif portatif d'administration de médicament selon la revendication 3, **caractérisé en ce que** la sortie (121) de ladite cartouche de médicament (12) est reliée à un cathéter pouvant être remplacé, et ledit moyen pour transférer (13, 14, 15) le médicament vers un utilisateur est conçu pour travailler dans un mode continu, de sorte que le médicament (123) est forcé hors de la cartouche (12) à travers la sortie dudit cathéter.

5. Dispositif portatif d'administration de médicament selon l'une quelconque des revendications 1 à 3, **caractérisé en ce que** ledit moyen pour transférer (13, 14, 15) une partie ou la totalité du médicament (123) en provenance de ladite cartouche de médicament (12) vers l'utilisateur comprend au moins une tige de piston (15) pouvant être mise en oeuvre pour mettre en prise et déplacer ladite paroi mobile (122), un moyen d'actionnement électriquement piloté (13), et un moyen d'entraînement (14) pour transférer le mouvement depuis ledit moyen d'actionnement électriquement piloté (13) vers ladite tige de piston (15).

6. Dispositif portatif d'administration de médicament selon l'une quelconque des revendications 1 à 5, **caractérisé en ce que** ledit moyen pour recevoir (16) les modules pouvant être remplacés comprend un moyen pour recevoir et fixer mécaniquement lesdits modules pouvant être remplacés au module de base, et un moyen pour électriquement relier (161) lesdits modules pouvant être remplacés (2, 3, 4, 5, 6) à un moyen électronique (31, 32) du module de base (1) et audit moyen pour fournir (17, 18) l'énergie électrique.

7. Dispositif portatif d'administration de médicament selon l'une quelconque des revendications 2 à 6, **caractérisé en ce que** le moyen électronique pour contrôler et commander (31, 32) le processus d'administration de médicament et pour communiquer avec des modules pouvant être remplacés et avec l'utilisateur est lui-même contenu dans un module pouvant être remplacé portatif.

8. Dispositif portatif d'administration de médicament selon l'une quelconque des revendications 2 à 7, **caractérisé en ce que** ledit moyen électronique (31, 32) comprend au moins un moyen pour commander une dose administrée pour commander le déplacement (33) de la paroi mobile (122) avec ladite tige de piston (15), par une commande du moyen d'actionnement électriquement piloté (13) par l'intermédiaire du moyen d'entraînement (14) pour transférer le mouvement depuis ledit moyen d'actionnement électriquement piloté (13) à ladite tige de piston (15), et un moyen pour contrôler le volume de médicament administré correspondant audit déplacement (33) de ladite paroi mobile (122), un moyen pour entrer (312) des données en provenance de l'utilisateur, un moyen de mémoire (313) pour stocker des données, un moyen pour communiquer (311, 32) avec les modules pouvant être remplacés (2, 3, 4, 5, 6), un moyen pour commander (314) la fonction du module de base et des modules pouvant être remplacés, un moyen de traitement (311) pour traiter des données d'entrée, pour traiter des données reçues en provenance des modules pouvant être remplacés et pour traiter des données stockées dans ledit moyen de mémoire, et un afficheur (315) pour visualiser lesdites données.

9. Dispositif portatif d'administration de médicament selon l'une quelconque des revendications 2 à 8, **caractérisé en ce que** ledit moyen électronique (31) comprend un moyen pour lire (316) un élément d'information sur une cartouche de médicament pouvant être remplacée (12) quand ladite cartouche est placée dans ledit moyen pour maintenir (11) une cartouche de médicament pouvant être remplacée, et un moyen pour traiter (311) ledit élément d'information.

10. Dispositif portatif d'administration de médicament selon l'une quelconque des revendications 2 à 9, **caractérisé en ce que** ledit moyen électronique est conçu pour recevoir une unité spécifique à l'utilisateur (314) contenant des données d'utilisateur, des procédures de vérification fonctionnelle et des procédures d'autorisation d'utilisateur.

11. Dispositif portatif d'administration de médicament selon la revendication 10, **caractérisé en ce que** ladite unité spécifique à l'utilisateur (314) est une carte à puce.

12. Dispositif portatif d'administration de médicament selon l'une quelconque des revendications 1 à 11, **caractérisé en ce que** les modules pouvant être remplacés peuvent être choisis à partir d'un groupe constitué par :
· un module pouvant être remplacé (201) contenant un système pour le contrôle du glucose dans le sang ;
· un module pouvant être remplacé (202) contenant un système pour mesurer continuellement le glucose dans le sang ;
· un module pouvant être remplacé (203) contenant un modem pour permettre la communication avec un réseau de communication de données ;
· un module pouvant être remplacé (204) contenant une interface de communication pour la communication sans fil avec d'autres dispositifs ;
· un module pouvant être remplacé (205) contenant des interfaces filaires pour communiquer avec un ou plusieurs d'un ordinateur individuel, d'un appareil de prise de vues, d'un écran de TV, d'un dispositif acoustique, d'un téléphone, d'un téléphone mobile ;
· un module pouvant être remplacé (206) contenant la fonctionnalité d'un téléphone mobile ;
· un module pouvant être remplacé (207) contenant un haut-parleur ;
· un module pouvant être remplacé (208) contenant un microphone, un haut-parleur et un processeur et un logiciel pour la reconnaissance de la parole pour fournir une interface vocale ;
· un module pouvant être remplacé (209) contenant un moyen pour contrôler la température de la cartouche de médicament et son contenu ;
· un module pouvant être remplacé (210) contenant un moyen pour contrôler et commander la température de la cartouche de médicament et son contenu ;
· un module pouvant être remplacé (211) contenant un moyen pour fournir un signal acoustique ou vibratoire ou optique pouvant être sélectionné après un certain temps pouvant être paramétré ou sur la survenance d'un certain événement ;
· un module pouvant être remplacé (212) contenant un moyen pour faire vibrer le contenu de la cartouche de médicament, et un moyen pour fournir un signal d'alarme indiquant l'écoulement d'un temps pouvant être paramétré pour assurer un mélange approprié des constituants de la cartouche de médicament ;
· un module pouvant être remplacé (213) contenant un moyen pour détecter des mouvements de tremblement du dispositif d'administration de médicament et un moyen pour fournir un signal d'alarme indiquant qu'une certaine quantité de mouvements de tremblement a été effectuée pour assurer un mélange approprié des constituants de la cartouche de médicament ;
· un module pouvant être remplacé (214) contenant un logiciel pour commander l'administration de médicament à des vitesses pouvant être fixées, des échelles de temps commandées, des doses maximales administrées, etc. ;
· un module pouvant être remplacé (215) contenant un logiciel pour produire un journal de certains événements définis par l'utilisateur contrôlés par le dispositif d'administration de médicament ;
· un module pouvant être remplacé (216) contenant un logiciel pour commander une ID d'utilisateur ;
· un module pouvant être remplacé (217) contenant un afficheur conçu pour une utilisation par un gaucher ;
· un module pouvant être remplacé (218) contenant un afficheur conçu pour une utilisation par un droitier ;
· un module pouvant être remplacé (219) contenant un moyen pour délivrer un profil de dose spécifique à un utilisateur à travers un cathéter en commandant ledit moyen pour transférer le médicament de sorte que l'on définit un mode continu de pompe.

13. Dispositif portatif d'administration de médicament selon l'une quelconque des revendications 1 à 12, **caractérisé en ce que** le module de base et les modules pouvant être remplacés sont munis de couvercles pouvant être remplacés (7, 8, 9).

14. Dispositif portatif d'administration de médicament selon l'une quelconque des revendications 1 à 13, **caractérisé en ce que** le module de base contient une fonctionnalité qui peut être verrouillée et rendue disponible à l'utilisateur seulement par une clé logicielle unique et/ou une mise à jour de logiciel.
